# FASCICULE DE BREVET EUROPEEN

(11) **EP 0 470 391 B1**
(45) Date de publication et mention de la délivrance du brevet: **23.08.1995**
(21) Numéro de dépôt: 91111607.7
(22) Date de dépôt: 12.07.1991
(51) Int. Cl.: C07D 213/06, A23L 1/226, A23L 1/235, A61K 7/48, A61K 7/46, C11B 9/00, C11D 3/50

(54) **Utilisation de pyridines à titre d'ingrédients parfumants et aromatisants**
Verwendung von Pyridinen als Parfüm- oder Aromazutaten
Use of pyridines as perfume or aroma ingredients

(30) Priorité: 10.08.1990 CH 2606/90
(43) Date de publication de la demande: 12.02.1992
(73) Titulaire: FIRMENICH SA, CH-1211 Genève 8 (CH)
(72) Inventeur: Thomas, Alan Francis, CH-1261 Borex (CH); Bassols, Frederico, CH-1213 Onex (CH)
(74) Mandataire: Salvadori, Giuseppe

(56) Documents cités:
- CH-A- 548 167
- GB-A- 1 156 483
- GB-A- 2 070 931
- US-A- 3 381 691
- US-A- 3 702 253
- JOURNAL OF AGRICULTURAL AND FOOD CHEMISTRY. vol. 29, no. 5, septembre 1981,WASHINGTON US pages 895 - 898; JOSEPH A. MAGA: ""Pyridines in foods""

## Description

La présente invention a trait au domaine des arômes et des parfums. Elle concerne plus particulièrement un procédé pour conférer, améliorer ou modifier les propriétés organoleptiques d'une composition parfumante ou aromatisante ou d'un article parfumé ou aromatisé, caractérisé en ce qu'on ajoute à ladite composition ou audit article un composé de formule 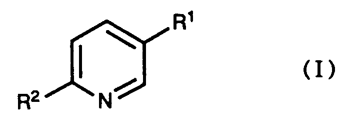
dans laquelle le symbole R¹ représente un radical alkyle saturé de chaîne linéaire, contenant de 6 à 8 atomes de carbone ou un radical 4-méthylhexyle et le symbole R² représente un atome d'hydrogène ou, dans le cas où R¹ contient 6 atomes de carbone, un atome d'hydrogène ou un radical méthyle.

A l'exception de la 3-(4-méthylhexyl)pyridine, pour laquelle nous n'avons trouvé aucune référence dans l'art antérieur, la structure chimique des composés de formule (I) est déjà connue. Cependant, nous n'avons trouvé dans l'art antérieur aucune mention des qualités organoleptiques de ces composés.

Nous avons maintenant découvert que les composés de formule (I) telle que définie ci-dessus possèdent des propriétés aromatisantes très intéressantes et qu'ils développent, à haute dilution, des notes gustatives très utiles pour l'aromatisation d'aliments et boissons, ainsi que d'autres produits de consommation traditionnellement aromatisés.

En effet, les composés (I) développent des notes aromatiques de type fruité, hespéridé qui peuvent être accompagnées d'arômes de type marin. C'est ainsi que la 3-hexylpyridine par exemple, développe un arôme fruité, hespéridé, rappelant l'arôme de l'écorce de fruit et la mandarine en particulier. Pour sa part, la 3-heptylpyridine possède un caractère gustatif moins fruité (rappelant toujours l'écorce de fruit), gras, métallique et accompagné d'une note de poisson. On y trouve aussi une note de type melon. La 8-octylpyridine possède elle une note fruitée, juteuse, accompagnée d'un arôme de fruits de mer et d'algues. D'autre part, la 5-hexyl-2-méthylpyridine possède un arôme gras, de poisson, métallique, accompagné d'une note rappelant la mandarine. Il s'agit de combinaisons de notes aromatiques tout à fait surprenantes et inattendues pour ce type de composés.

On connaît de l'art antérieur, notamment des brevets US-A-3'669'908 et US-A-3'716'543, des pyridines substituées à chaîne alkylénique ou alkadiénique, qui possèdent des notes aromatiques réminiscentes des arômes de fruits de mer. Il est décrit également dans les références citées que ces pyridines confèrent aux compositions aromatiques dans lesquelles elles sont incorporées des notes de type marin, "bord de mer", "fruits de mer", épicées ou encore boisées-ambrées.

D'autre part, on connaît également d'après le brevet US-A-3'702'253 des pyridines à chaîne alkylique ou alkylénique typiques de l'arôme du café, possédant des notes aromatiques où les caractères amer, vert, rôti, astringent et noisette sont fortement représentés (voir aussi GB-A-1 156 483).

D'autres références de la littérature scientifique font état, par exemple, de pyridines substituées à chaîne alkylique utiles pour l'aromatisation du tabac [voir, par exemple, US-A-3'381'691], ou encore particulièrement adaptées à la préparation de compositions parfumantes, mais présentant également un intérêt pour l'industrie des arômes [voir GB-A-2 070 931].

CH-A-548167 divulgue l'utilisation de certaines pyrazines, ou de mélanges de celles-ci avec la 2-éthylpyridine, la 2-acétyl-3-hydroxypyridine et/ou la 3-n-propylpyridine, pour impartir à des produits alimentaires des notes gustatives de type rôti ou brûlé.

D'autre part, J. A. Maga dans J. Agr. Food Chem. 29 (1981), 895-898 passe en revue les propriétés organoleptiques d'un grand nombre de pyridines, et notamment de plusieurs alkylpyridines, couramment utilisées dans l'aromatisation d'aliments. L'auteur indique que la plupart de ces composés possèdent des notes organoleptiques de type vert, amer, astringent, rôti ou brûlé et cite, en particulier, que la 2-pentylpyridine, en solution diluée, développe une odeur à caractère gras et de suif.

Cependant, aucun de ces composés connus ne possède la combinaison de notes aromatiques décrites plus haut que l'on retrouve dans les pyridines de formule (I) utilisées selon la présente invention et qui rend les composés de l'invention particulièrement utiles aussi bien dans des applications de type fruité, notamment hespéridé, que dans des applications de type salé, comme il ressort des exemples présentés plus loin.

Notre invention est basée sur la découverte fortuite que les pyridines de formule (I) étaient des constituants de l'huile essentielle d'orange. Leur présence, jusqu'ici insoupçonnée, a été décelée grâce à l'emploi d'une technique fort complexe, faisant appel à des méthodes utilisant la chromatographie en phase gazeuse. Il s'agit de composés naturels dont la présence dans l'huile essentielle d'orange avait échappé jusqu'ici aux nombreux examens auxquels avait été soumise cette huile.

Ces composés ont été extraits d'une d'huile d'orange obtenue par pression à froid d'oranges de Floride.

L'huile (100 kg) a été extraite sous agitation et N₂ avec de l'acide sulfurique (aqueux à 10%, 2x1 l) et ensuite avec de l'eau (2x1 l). On a laissé agiter pendant 4 min et décanté pendant environ 2 h entre chaque opération et, pour éliminer les émulsions obtenues dans la phase aqueuse, on a utilisé une super cellule Hyflow et filtré sur Büchner avant d'extraire. Les phases acide et aqueuse combinées ont été lavées au pentane (3x500 ml, en utilisant du pentane commercial très pur), ensuite alcalinisées avec du carbonate de sodium solide. La solution alcalinisée a été extraite au pentane (3x200 ml). Cette phase organique a été séchée sur MgSO₄ et concentrée (40°/13x10³ Pa), pour fournir environ 280 mg de produit. Celui-ci a été soumis à une nouvelle extraction par dilution dans 20 ml d'acétate d'éthyle, suivie d'extraction avec H₂SO₄ (2x∼ 7 ml) et H₂O (2x∼ 7 ml). La phase aqueuse a été alcalinisée avec du carbonate de sodium solide et extraite avec de l'acétate d'éthyle (3x10 ml). Après séchage sur MgSO₄ et concentration, on a obtenu environ 8 mg d'extrait, constitué essentiellement de produits azotés. Cet extrait a été directement injecté dans un appareil de couplage CG-SM (chromatographie en phase gazeuse - spectrométrie de masse).

Des pyridines décelées par ce procédé, ainsi que leurs temps de rétention dans deux types de colonne et leurs spectres de masse, sont présentés ci-après :

| Composé | Temps de rétention/min | |
|---|---|---|
| | Colonne Supelcowax 10 15m | Colonne SPB-1 60m |
| 1. 3-hexylpyridine | 10,20 | 22,29 |
| 2. 3-heptylpyridine | 11,99 | 25,39 |
| 3. 3-octylpyridine | 15,50 | 28,38 |
| 4. 3-phénylpyridine | 18,15 | 25,10 |
| 5. 5-hexyl-2-méthylpyridine | 11,16 | 24,14 |
| 6. 3-(4-méthylhexyl)pyridine | 10,39 | 27,59 |
| 7. 2-méthyl-5-phénylpyridine | 18,37 | 30,66 |
| 8. 3-(4-méthyl-1-phényl)pyridine | 19,80 | 32,09 |
| Spectres de masse : 1. 163(10,M⁺), 162(10), 148(3), 134(9), 120(33), 107(14), 106(85), 93(100), 92(70) 2. 177(4,M⁺), 162(2), 148(6), 134(13), 120(15), 107(15), 106(75), 93(100), 92(40) 3. 191(11,M⁺), 162(8), 148(14), 134(7), 120(18), 107(19), 106(98), 93(100), 92(40) 4. 155(100,M⁺), 154(53), 128(11), 127(15), 126(7), 102(12) 5. 177(10,M⁺), 176(5), 162(2), 148(4), 134(15), 120(38), 107(35), 106(100), 93(4), 92(3) 6. 177(10,M⁺), 176(8), 162(9), 148(30), 134(5), 120(30), 107(33), 106(71), 93(100), 92(88), 65(26), 57(25), 43(28), 41(24), 39(15) 7. 169(100,M⁺), 168(20), 167(10), 154(4), 141(23), 127(7), 115(15), 102(12), 85(7), 77(8), 63(9) 8. 169(100,M⁺), 168(60), 167(18), 154(6), 141(10), 126(4), 115(14), 91(9), 84(7), 71(13), 63(7) | | |

La structure des pyridines de formule (I), déduite à partir de leur spectre de masse indiqué ci-dessus, a été confirmée par synthèse. Les méthodes utilisées pour la synthèse de ces composés (I) sont décrites plus loin.

Malgré les différentes études effectuées dans le passé sur l'huile d'orange, qui ont permis d'identifier plus de 200 constituants [voir dans "Volatile Compounds in Foods - Qualitative and Quantitative Data", vol. I, pages 44-49, ed. TNO, Hollande (1989) et références y-citées], aucune pyridine n'avait jusqu'ici été décelée dans cette huile. Nous avons maintenant découvert, entre autres, celles mentionnées plus haut, qui y sont présentes dans des quantités extrêmement faibles, de l'ordre de 1 ppm. Parmi ces pyridines, il est apparu à l'évidence que les composés de formule (I) selon l'invention contribuaient de façon déterminante à l'arôme typique de l'orange et qu'ils pouvaient de ce fait servir à la reconstitution de l'essence de ce fruit, lorsqu'utilisés à haute dilution.

Les composés de l'invention apportent donc une solution originale au problème de la reconstitution de l'arôme spécifique de l'orange. A cet effet, il y a lieu de remarquer que leur présence dans l'huile essentielle d'orange était tout à fait inattendue et qu'il aurait été impossible de prévoir que ces pyridines pouvaient développer, à haute dilution, des notes aromatiques tout à fait essentielles à la reproduction du caractère gustatif naturel du fruit même, et de son jus en particulier.

Les propriétés organoleptiques de ces composés sont telles, cependant, que leur champ d'application est bien plus varié que celui qui serait défini par la seule reconstitution de l'essence naturelle de l'orange. Ils trouvent, en effet, une utilisation fort étendue dans le domaine des arômes, où ils servent à conférer, développer ou améliorer la note typique des agrumes, tout particulièrement celle de l'orange. Par exemple, ils confèrent à l'essence d'orange de la puissance et un goût plus juteux et plus typique du zeste de fruit. Ils peuvent être utilisés dans d'autres applications de type citrus, telles que des arômes de type citron, pamplemousse, citron vert, mandarine ou tangerine.

Par ailleurs, ils trouvent également un emploi fort avantageux dans un domaine d'aromatisation complètement différent, celui des aliments salés et épicés, dans lesquels ils servent à développer ou à renforcer le caractère gustatif de poisson ou de fruits de mer de l'aliment.

Pour produire les effets aromatisants désirés, les pyridines (I) sont utilisées de préférence à des concentrations très faibles. C'est ainsi que des effets intéressants ont été obtenus avec des concentrations de l'ordre de 20 ppb de pyridine (I), par rapport au poids total de la composition dans laquelle elle est incorporée. Ces concentrations peuvent aller jusqu'à 5 ppm, suivant le type d'application.

Les composés de l'invention se prêtent à l'aromatisation d'articles divers tels les aliments, les boissons, les chewing-gums, les dentifrices ou encore les préparations pharmaceutiques.

A titre d'aliment pouvant être aromatisé, on peut mentionner les glaces, les crèmes à dessert, les yogourts, les produits lactés en général, les produits de confiserie ou de boulangerie, les sirops, les sucres cuits, les confitures ou, encore, les soupes et bouillons, les extraits pour la préparation de soupes et de sauces ou, en général, les produits à base de poisson ou de fruits de mer, ou cherchant à imiter ces caractères gustatifs. On peut citer également les aliments du type amuse-gueules, chips ou autres.

Les composés de formule (I) sont incorporés aux aliments, boissons, chewing-gums, dentifrices ou préparations pharmaceutiques que l'on désire aromatiser selon des procédés usuels dans l'art, soit seuls, soit en mélange avec d'autres ingrédients aromatisants naturels ou synthétiques. Ils peuvent être employés tels quels ou en solution dans l'un des solvants comestibles usuels tels la triacétine, l'alcool éthylique ou le propylène glycol, ou en mélange sur un support solide, par exemple, une dextrine ou la gomme arabique.

Les pyridines (I) selon l'invention présentent également un intérêt pour la parfumerie. Par exemple, la 3-heptylpyridine, que l'on cite à titre préférentiel, développe une note odorante rappelant celle de l'α-sinensal, ou 2,6,10-triméthyl-2,6,11-dodécatrién-1-al, avec un caractère mandarine et une connotation fraîche. Ce composé confère aux compositions dans lesquelles il est incorporé une odeur naturelle rappelant la fleur d'oranger.

Un autre ingrédient parfumant préféré selon l'invention est la 3-hexylpyridine qui possède une note odorante fruitée et grasse, rappelant l'orange.

Les pyridines (I) peuvent être utilisées avantageusement, aussi bien en parfumerie fine que dans des applications fonctionnelles, pour la préparation de compositions parfumantes et articles parfumés. Parmi ces derniers on peut citer les parfums ou eaux de toilette, les savons, les gels de douche ou bain, les shampoings et autres produits d'hygiène capillaire, les préparations cosmétiques et les désodorisants corporels ou d'air ambiant. Elles trouvent également un emploi utile dans le parfumage de détergents, d'adoucissants textiles ou de produits d'entretien.

Lorsqu'elles sont utilisées pour ces applications, des effets olfactifs intéressants peuvent être obtenus en utilisant de faibles concentrations, typiquement de l'ordre de 0,01 à 0,2% en poids par rapport au poids de composition dans laquelle elles sont incorporées.

Les composés de formule (I) monosubstitués, à savoir les 3-alkylpyridines, ont été préparés selon une méthode générale décrite ci-après pour le cas particulier de la 3-hexylpyridine.

Une solution d'amidure de sodium dans de l'ammoniac liquide a été préparée en ajoutant de petits morceaux de sodium à environ 300 ml d'ammoniac liquide contenant 0,1 g de nitrate ferrique, jusqu'à addition de 5,78 g de sodium au total. Une fois la réaction terminée, on a ajouté 20,48 g de 3-méthylpyridine à -30°C (10 min), et ensuite 36,24 g (29,8 ml) de bromure de pentyle, sur 15 min. Le mélange a été agité pendant encore 15 min, ensuite on a laissé évaporer l'ammoniac et ajouté de l'eau glacée pour compléter l'hydrolyse. Le mélange a été extrait à l'éther (2 fois) et cet extrait a été lavé avec de l'eau et de nouveau extrait avec HCl (4 fois, aqueux à 10%) et une fois avec de l'eau. Les phases aqueuses combinées ont été rendues basiques à l'aide d'une solution aqueuse à 20% d'hydroxyde de sodium, à 0°C. Après extrachon à l'éther, on a lavé avec de l'eau, séché et concentré pour obtenir 23 g de produit brut. Ce dernier a été distillé une première fois sur colonne Vigreux pour fournir 17 g de 3-hexylpyridine (P. éb. 65-67°C/13 Pa). Le produit a été purifié davantage à l'aide d'une deuxième distillation sur colonne Vigreux pour fournir la 3-hexylpyridine pure à au moins 99,95% (rend. : 47%).

Les autres 3-alkylpyridines ont été préparées de façon similaire, en utilisant le bromure d'alkyle approprié. Les données analytiques de ces composés synthétisés étaient les suivantes.

### 3-hexylpyridine

Temps de rétention (sur colonne non polaire SPB-1) : 22,29 min
RMN(¹H,360MHz) : 0,88(t,3H) ; 1,2-1,4(m,6H) ; 1,54-1,66(m,2H) ; 2,59(t,2H) ; 7,19(dxd,1H) ; 7,49(d,1H) ; 8,44(superposé sur 8,43, 2H) δ ppm
SM : 163(10,M⁺), 162(10), 148(3), 134(9), 120(33), 107(14), 106(85), 93(100), 92(70).
Seuil de perception : 0,28 ppb

### 3-heptylpyridine

Temps de rétention (sur colonne non polaire SPB-1) : 25,39 min
P. éb. : 72-74°C/13 Pa
RMN(¹H,360MHz) : 0,88(t,3H) ; 1,2-1,4(m,8H) ; 1,54-1,66(m,2H) ; 2,59(t,2H) ; 7,19(dxd,1H) ; 7,49(d,1H) ; 8,44(superposé sur 8,43, 2H) δ ppm
SM : 177(4,M⁺), 162(2), 148(6), 134(13), 120(15), 107(15), 106(75), 93(100), 92(40).

### 3-octylpyridine

Temps de rétention (sur colonne non polaire SPB-1) : 28,38 min
P. éb. : 108°C/13 Pa
RMN(¹H,360MHz) : 0,88(t,3H) ; 1,2-1,4(m,10H) ; 1,54-1,66(m,2H) ; 2,59(t,2H) ; 7,19(dxd,1H) ; 7,49(d,1H) ; 8,44(superposé sur 8,43, 2H) δ ppm
SM : 191(11,M⁺), 162(8), 148(14), 134(7), 120(18), 107(19), 106(98), 93(100), 92(40).
La 5-hexyl-2-méthylpyridine a été préparée à partir du n-octanal selon la méthode suivante.
Dans un ballon à 3 cols, on a chargé 100 g (0,78 mmole) de n-octanal, 73 g d'aldéhyde formique à 36% et 260g (0,78 mole) de catalyseur (chlorhydrate de diéthylamine aqueux ; 3 mole/kg). On a chauffé le mélange à reflux durant 1 h et distillé. Le produit de la réaction a été extrait à l'éther, séché et concentré pour fournir 42,41 g de 2-hexyl-2-propénal. L'extraction du résidu du ballon a fourni encore 64 g de produit brut. L'aldéhyde a été utilisé tel quel dans la poursuite de la synthèse.
A une suspension d'iodure de zinc (0,21 g ; 0,671 mmole) dans 42 g (0,3 mole) de 2-hexyl-2-propénal, on a ajouté goutte à goutte, à 60°C et sous azote, 18,5 g (0,25 mole) de 2-méthoxypropène. Une fois l'introduction finie (23 min), on a laissé la réaction se poursuivre pendant 6 h à 60°C et ensuite distillé sur colonne Vigreux pour obtenir 8,16 g de 5-hexyl-3,4-dihydro-2-méthoxy-2-méthyl-2H-pyrane (p. éb. 73-74°C/3 Pa : rend. 15,4%) qui a été utilisé dans la réaction suivante.
A une solution contenant 2,76 g (39,47 mmole) d'hydrochlorure d'hydroxylamine, 8,58 g (143,10 mmole) d'acide acétique et 692 mg (38,49 mmole) d'eau, on a ajouté goutte à goutte, à 100°C et sous azote, sur 3 h, 8,16 g (38,49 mmole) de l'hydroxypyrane obtenu dans la réaction précédente.
On a agité encore 30 min à 100°C, refroidi le mélange réactionnel et versé sur une solution de NaOH et eau glacée. On a extrait la solution aqueuse trois fois à l'éther et lavé la solution éthérée quatre fois avec HCl à 10% et ensuite une fois à l'eau (sel). La solution aqueuse a été alcalinisée à 0°C avec NaOH à 20%. Après une nouvelle extraction à l'éther (2 fois), on a lavé à l'eau, séché, concentré et distillé au four à boules (130°-150°C/3 Pa) pour obtenir 4,187 g (rend. 62,2%) de 5-hexyl-2-méthylpyridine dont les données analytiques étaient les suivantes :
RMN(¹H,360MHz) : 0,88(t,3H) ; 2,47(s,3H) ; 2,56(t,3H) ; 7,06(d,J=5Hz,1H) ; 7,37(dxd, J=5, 2Hz,1H) ; 8,31(d,J=2Hz,1H) δ ppm
RMN(¹³C) : 14,0(q) ; 22,6(t) ; 23,9(q) ; 28,9(t) ; 31,2(t) ; 31,7(t) ; 32,7(t) ; 122,8(d) ; 134,8(s) ; 136,2(d) ; 149,2(d) ; 155,6(s) δ ppm.
SM : 177(10,M⁺), 176(5), 162(2), 148(4), 134(15), 120(30), 107(35), 106(100), 93(3), 79(13), 77(19).
La 3-(4-méthylhexyl)pyridine a été préparée de façon similaire à celle décrite plus haut pour la 3-hexylpyridine mais en utilisant du bromure de 3-méthyl-1-pentyle [préparé en ajoutant pendant 1 h, à -30°C et sous N₂, 22,5 g de PBr₃ (0,083 mole; 7,8 ml) à un mélange de 21,6 g (0,212 mole) de 3-méthyl-1-pentanol, 3,95g de pyridine anhydre et 88,4 ml d'éther sulfurique anhydre; on a laissé réagir 1 h à -30°C, ensuite une nuit à température ambiante; le produit de la réaction a été extrait et lavé de façon habituelle et purifié sur colonne Vigreux pour fournir 20,8g de produit pur].
On a utilisé 300 ml d'ammoniac liquide, 4,62 g (0,201 mole) de Na, 7,1 g (76 mmole) de 3-méthylpyridine et 14 g (84 mmole) de bromure de 3-méthyl-1-pentyle.
Les données analytiques de la 3-(4-méthylhexyl)pyridine étaient les suivantes :
RMN(¹H,360MHz,CDCl₃) : 0,85(t,J=7Hz,superimposé sur d,J=7Hz,6H) ; 1,05-1,21(m,2H) ; 1,25-1,40(m,3H) ; 1,50-1,70(m,2H) ; 2,58(t,J=7Hzxd, J=1Hz, 2H) ; 7,20(dxd,1H) ; 7,49(d,1H) ; 8,44(d) superimposé sur 8,45(s) (2H ensemble) δ ppm
SM : 177(10,M⁺), 176(8), 162(9), 148(30), 134(5), 120(30), 107(33), 106(71), 93(100), 92(88), 65(26), 57(25), 43(28), 41(24), 39(15).
Cette pyridine développait un arôme gras, vert, juteux et de noisette.

L'invention sera maintenant décrite en détail à l'aide des exemples présentés ci-après.

### Exemple 1

### Application dans l'huile essentielle d'orange

On a préparé cinq échantillons d'huile essentielle d'orange avec les ingrédients suivants :

| | Echantillon | | | | |
|---|---|---|---|---|---|
| Ingrédient | A | B | C | D | E |
| | % en poids | | | | |
| Huile essentielle d'orange de Floride | 100,0000 | 99,9975 | 99,9950 | 99,9950 | 99,9800 |
| 3-hexylpyridine | - | 0,0025 | - | - | - |
| 3-heptylpyridine | - | - | 0,0050 | - | - |
| 3-octylpyridine | - | - | - | 0,0050 | - |
| 5-hexyl-2-méthylpyridine | - | - | - | - | 0,0200 |
| Total | 100,0000 | 100,0000 | 100,0000 | 100,0000 | 100,0000 |

Un panel d'experts aromaticiens a évalué les 5 échantillons A, B, C, D et E dans une solution acide (10% sucre, 0,1% acide citrique) à 0,1% dans l'eau de source, à laquelle on avait ajouté 300 ppm d'échantillon d'huile d'orange.
De l'avis des experts, l'échantillon A avait un goût doux, aldéhydique, typique de l'écorce d'orange. L'échantillon B avait un caractère plus juteux et avait plus de volume que l'huile essentielle A. Le goût de cet échantillon était aussi plus éclatant et avait plus d'impact, son caractère étant aussi plus aldéhydique.
L'échantillon C a lui été trouvé plus doux, avec un caractère plus juteux et plus zeste que celui de l'huile A. Il avait aussi plus de volume et avait un caractère légèrement gras.
Quant à l'échantillon D, les experts l'ont trouvé plus juteux et avec plus de caractère de zeste que l'échantillon A. Il était aussi plus ample, plus copieux.
Par ailleurs, il possédait une note aldéhydique très propre et un peu de caractère mandarine.
L'échantillon E a été jugé plus juteux, avec un caractère écorce plus prononcé que l'échantillon A. Il avait également un goût plus arrondi, volumineux et pulpeux que ce dernier et était plus doux et moins terpénique.

### Exemple 2

### Composition aromatique de type salé

On a préparé une composition aromatique de base de type salé, surimi, par mélange des ingrédients suivants :

| Ingrédients | Poids (g) |
|---|---|
| Surimi | 400 |
| Sel | 10 |
| Eau gelée | 136 |
| Sucre | 8 |
| Amidon (pomme de terre) | 20 |
| MSG* | 2 |
| Extrait de crevette | 4 |
| Extrait de levure | 2 |
| Poudre d'albumine | 20 |
| Total | 6̅0̅2̅ |

| | |
|---|---|
| * monosodium glutamate | |

Avec cette composition surimi de base, on a préparé trois compositions aromatiques A, B et C en ajoutant à la base surimi respectivement 50 ppb de 3-hexylpyridine, 200 ppb de 3-heptylpyridine et 200 ppb de 3-octylpyridine.
La composition de base et les trois nouvelles compositions A, B et C ainsi aromatisées ont été chauffées à 90°C pendant 30 min, refroidies et ensuite congelées.
Un panel d'experts aromaticiens a évalué les trois compositions A, B et C après leur décongélation et les a comparées avec la composition de base chauffée de façon identique. De l'avis des experts, la composition surimi de base représentait un goût de poisson assez fade et doux, alors que la composition A était plus douce et plus propre que la base surimi. Sa note de type "fruits de mer" était aussi renforcée, plus ample et avait plus d'impact.
Le caractère poisson de la composition B a été jugé plus intense que celui de la composition de base. Il était aussi plus gras et son caractère de type crevette sortait renforcé. La composition B avait aussi plus d'impact que la composition de base.
Quant à la composition C, elle a également été jugée meilleure que la composition de base, car son goût était aussi plus poissonneux et plus viandeux. De plus, les notes de crabe étaient renforcées et la composition C avait plus de volume que la base surimi.

### Exemple 3

### Application dans un jus d'orange commercial

On a préparé quatre échantillons de jus d'orange à partir d'un jus d'orange de base obtenu par simple dilution d'un concentré congelé d'origine commerciale, auquel on a ajouté 25 ppb de 3-hexylpyridine (composition A), 50 ppb de 3-heptylpyridine (composition B), 50 ppb de 3-octylpyridine (composition C) et 500 ppb de 5-hexyl-2-méthylpyridine (composition D).
Les quatre compositions A, B, C et D ont été évaluées par un panel d'experts aromaticiens et comparées avec le jus d'orange de base qui possédait un goût doux et "cuit" typique.
De l'avis des experts, la composition A possédait un goût plus vert et plus frais que le jus d'orange de base. Elle avait aussi un impact juteux plus fort et réussissait à couvrir le caractère "cuit" que l'on retrouvait dans le jus de base. La composition B avait plus de volume, également un goût plus doux et juteux que le jus de base. Elle était légèrement grasse et verte, et plus onctueuse que le jus de base, dont la note "cuite" était également couverte.
La composition C, pour sa part, était plus douce et fruitée, la note fruitée ayant plus de volume que le jus de base. Elle était également plus juteuse et verte.
Finalement, la composition D était plus douce et juteuse que le jus de base et possédait aussi plus de volume. Son caractère gustatif était aussi moins "cuit" et plus frais.

### Exemple 4

### Préparation d'une composition parfumante

On a préparé une composition parfumante de base par mélange des ingrédients suivants :

| Ingrédients | Poids (g) |
|---|---|
| Géraniol | 15,0 |
| Phénéthylol | 5,0 |
| Linalol | 31,5 |
| HEDIONE ® ¹⁾ | 10,0 |
| Anthranilate de méthyle | 2,5 |
| Essence de petitgrain | 10,0 |
| Acétate de géranyle | 5,0 |
| α-Terpinéol | 5,0 |
| Essence de fleur d'oranger | 15,0 |
| Total | 9̅9̅,̅0̅ |

| | |
|---|---|
| 1) dihydrojasmonate de méthyle ; origine : Firmenich SA, Genève, Suisse | |

Lorsqu'on a ajouté à cette composition de base de type hespéridé 0,1% en poids, par rapport au poids total de la composition, de 3-heptylpyridine, on a obtenu une nouvelle composition qui développait une note sèche, amère et rafraîchissante rappelant une partie de l'odeur de l'absolue de la fleur d'oranger.

### Exemple 5

### Préparation d'une composition parfumante

On a préparé une composition parfumante de base par mélange des ingrédients suivants :

| Ingrédients | Poids (g) |
|---|---|
| Acétate de terpényle | 15,0 |
| Géraniol | 15,0 |
| Linalol | 10,0 |
| Phénéthylol | 15,0 |
| Eugénol | 5,0 |
| L-carvone | 5,0 |
| Anéthole | 2,5 |
| Acétate de 4-(1,1-diméthyléthyl)-1-cyclohexyle ¹⁾ | 15,0 |
| Essence de violette | 2,5 |
| Lilial ® ²⁾ | 5,0 |
| cis-3-Hexénol à 10%* | 1,5 |
| Total | 9̅1̅,̅5̅ |

| | |
|---|---|
| * dans le dipropylèneglycol | |
| 1) mélange d'isomères cis:trans ; origine : Firmenich SA, Genève, Suisse | |
| 2) 3-(4-tert-butyl-1-phényl)-2-méthylpropanal ; origine : L. Givaudan, Vernier, Suisse | |

Lorsqu'on a ajouté à cette composition de base 0,1% en poids, par rapport au poids de la composition, de 3-heptylpyridine, on a obtenu une nouvelle composition qui développait une note plus fraîche et diffuse et moins douce que celle de la composition de base. Le composé de l'invention avait produit un effet odorant de type vert, herbacé et fleuri, en rehaussant le caractère épicé, menthé et violette de la composition de base.
Le même type d'effet, bien que légèrement moins prononcé, a été observé lorsqu'on a ajouté à la composition de base 0,1% en poids de 3-hexylpyridine.

## Revendications

1. Procédé pour conférer, améliorer ou modifier les propriétés organoleptiques d'une composition parfumante ou aromatisante ou d'un article parfumé ou aromatisé, caractérisé en ce qu'on ajoute à ladite composition ou audit article un composé de formule 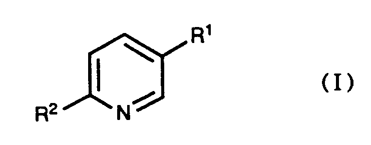 dans laquelle le symbole R¹ représente un radical alkyle saturé de chaîne linéaire, contenant de 6 à 8 atomes de carbone ou un radical 4-méthylhexyle et le symbole R² représente un atome d'hydrogène ou, dans le cas où R¹ contient 6 atomes de carbone, un atome d'hydrogène ou un radical méthyle.

2. Composition parfumante ou article parfumé contenant à titre d'ingrédient actif un composé de formule (I) définie à la revendication 1.

3. Composition parfumante ou article parfumé selon la revendication 2 contenant à titre d'ingrédient parfumant la 3-hexylpyridine ou la 3-heptylpyridine.

4. Article parfumé selon la revendication 2 ou 3, sous forme d'un parfum ou d'une eau de toilette, d'un savon, d'un gel de douche ou bain, d'un shampoing ou autre produit d'hygiène capillaire, d'une préparation cosmétique, d'un désodorisant corporel ou d'air ambiant, d'un détergent ou adoucissant textile ou d'un produit d'entretien.

5. Composition aromatisante ou article aromatisé contenant à titre d'ingrédient actif un composé de formule (I) définie à la revendication 1, dans sa forme pratiquement pure et non accompagné des substances d'origine naturelle présentes dans l'huile essentielle d'orange.

6. Composition aromatisante ou article aromatisé selon la revendication 5, caractérisé en ce que la concentration du composé de formule (I) est comprise entre environ 20 ppb et 5 ppm du poids total de la composition ou de l'article.

7. Procédé pour conférer, améliorer ou modifier le caractère gustatif de type hespéridé d'une composition aromatisante ou d'un article aromatisé , caractérisé en ce qu'on ajoute à ladite composition ou audit article un composé de formule (I) définie à la revendication 1.

8. Procédé selon la revendication 7, caractérisé en ce qu'on ajoute le composé de formule (I) à une concentration comprise entre environ 20 ppb et 5 ppm du poids total de la composition ou de l'article.

9. Composition aromatisante ou article aromatisé à caractère hespéridé contenant, à titre d'ingrédient aromatisant, un composé de formule (I) définie à la revendication 1, dans sa forme pratiquement pure et non accompagné des substances d'origine naturelle présentes dans l'huile essentielle d'orange.

10. Composition aromatisante ou article aromatisé selon la revendication 9, caractérisé en ce que la concentration du composé de formule (I) est comprise entre 20 ppb et 5 ppm du poids total de la composition ou de l'article.

11. Procédé pour améliorer le caractère gustatif propre de l'orange dans un article tel qu'un aliment, une boisson, un chewing gum, un dentifrice ou une préparation pharmaceutique, caractérisé en ce qu'on ajoute audit article un composé de formule (I) définie à la revendication 1, dans une concentration comprise entre 20 ppb et 5 ppm du poids total de l'article.

12. 3-(4-Méthylhexyl)pyridine.

## Claims

1. A process to confer, improve or modify the organoleptic properties of a perfuming or flavouring composition, or of a perfumed or flavoured article, characterised in that there is added to said composition or article a compound of formula 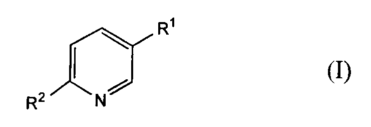 wherein R¹ stands for a saturated linear alkyl radical having 6 to 8 carbon atoms, or for a 4-methylhexyl radical, and R² represents either a hydrogen atom or, when R¹ has 6 carbon atoms, a hydrogen atom or a methyl radical.

2. A perfuming composition or a perfumed article containing as active ingredient a compound of formula (I) as defined in claim 1.

3. A perfuming composition or a perfumed article according to claim 2, containing as perfuming ingredient 3-hexylpyridine or 3-heptylpyridine.

4. A perfumed article according to claim 2 or 3, in the form of a perfume or cologne, a soap, a bath or shower gel, a shampoo or any other hair-care product, a cosmetic preparation, a body or air deodorant, a detergent or fabric softener, or a household product.

5. A flavouring composition or a flavoured article containing as active ingredient a compound of formula (I) as defined in claim 1, in its practically pure form and non-accompanied by the natural origin substances present in orange essential oil.

6. A flavouring composition or a flavoured article according to claim 5, characterised in that the concentration of compound of formula (I) is comprised between about 20 ppb and 5 ppm of the total weight of said composition or article.

7. A process to confer, improve or modify the citrus-type flavour character of a flavouring composition or a flavoured article, characterised in that there is added to said composition or article a compound of formula (I) as defined in claim 1.

8. A process according to claim 7, characterised in that the compound of formula (I) is added in a concentration comprised between about 20 ppb and 5 ppm of the total weight of said composition or article.

9. A flavouring composition or a flavoured article, having a citrus-type character, containing as flavouring ingredient a compound of formula (I) as defined in claim 1, in its practically pure form and non-accompanied by the natural origin substances present in orange essential oil.

10. A flavouring composition or a flavoured article according to claim 9, characterised in that the concentration of the compound of formula (I) is comprised between about 20 ppb and 5 ppm of the total weight of said composition or article.

11. A process to improve the orange-type flavour character in an article such as a foodstuff, a beverage, a chewing-gum, a toothpaste or a pharmaceutical preparation, characterised in that there is added to said article a compound of formula (I) as defined in claim 1, in a concentration comprised between about 20 ppb and 5 ppm of the total weight of the article.

12. 3-(4-Methylhexyl)pyridine.

## Patentansprüche

1. Verfahren zum Verleihen, Verbessern oder Verändern von organoleptischen Eigenschaften einer Riechstoff- oder Aromakomposition oder eines parfümierten oder aromatisierten Artikels, dadurch gekennzeichnet, dass man zu der genannten Komposition oder dem genannten Artikel eine Verbindung der Formel 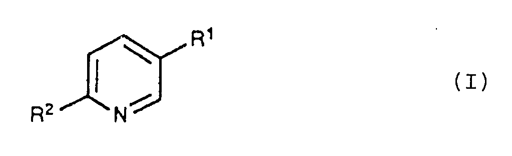 worin das Symbol R¹ einen geradkettigen, gesättigten Alkylrest mit 6 bis 8 Kohlenstoffatomen oder einen 4-Methylhexylrest bedeutet und das Symbol R² für ein Wasserstoffatom oder, falls R¹ 6 Kohlenstoffatome besitzt, für ein Wasserstoffatom oder einen Methylrest steht, hinzufügt.

2. Riechstoffkomposition oder parfümierter Artikel, enthaltend als Wirkstoff eine Verbindung der Formel (I), welche im Anspruch 1 definiert ist.

3. Riechstoffkomposition oder parfümierter Artikel gemäss Patentanspruch 2, enthaltend als Riechstoffbestandteil 3-Hexylpyridin oder 3-Heptylpyridin.

4. Parfümierter Artikel gemäss Patentanspruch 2 oder 3, in Form eines Parfüms oder eines Toilettwassers, einer Seife, eines Douche- oder Badegels, eines Shampoos oder eines anderen Mittels der Haarpflege, einer kosmetischen Zubereitung, eines Körperdesodorants oder eines Raumluftverbesserers, eines Detergenz oder eines Textilauffrischungsmittels oder eines Pflegeproduktes.

5. Aromakomposition oder aromatisierter Artikel, enthaltend als Wirkstoff eine Verbindung der Formel (I), welche im Anspruch 1 definiert ist, in ihrer praktisch reinen und von natürlichen Verbindungen, welche im essentiellen Orangenöl vorkommen, nicht begleiteten Form.

6. Aromakomposition oder aromatisierter Artikel gemäss Anspruch 5, dadurch gekennzeichnet, dass die Konzentration der Verbindung der Formel (I) zwischen etwa 20 ppb und 5 ppm des Gesamtgewichtes der Komposition oder des Artikels beträgt.

7. Verfahren zum Verleihen, Verbessern oder Verändern des Geschmackscharakters vom Hesperidentypus einer Aromakomposition oder einer aromatisierten Verbindung, dadurch gekennzeichnet, dass man zu der genannten Komposition oder dem genannten Artikel eine Verbindung der Formel (I), welche im Anspruch 1 definiert ist, zusetzt.

8. Verfahren gemäss Anspruch 7, dadurch gekennzeichnet, dass man die Verbindung der Formel (I) in einer Konzentration zwischen etwa 20 ppb und 5 ppm des Gesamtgewichtes der Komposition oder des Artikels zusetzt.

9. Aromakomposition oder aromatisierter Artikel mit hesperidenartigem Charakter, enthaltend als aromatisierenden Bestandteil eine Verbindung der Formel (I), welche im Anspruch 1 definiert ist, in ihrer praktisch reinen und von natürlichen Substanzen, welche im essentiellen Orangenöl vorkommen, nicht begleiteten Form.

10. Aromakomposition oder aromatisierter Artikel gemäss Anspruch 9, dadurch gekennzeichnet, dass die Konzentration der Verbindung der Formel (I) zwischen 20 ppb und 5 ppm des Gesamtgewichtes der Komposition oder des Artikels beträgt.

11. Verfahren zum Verbessern des der Orange eigenen Geschmackscharakters in einem Artikel, wie einem Nahrungsmittel, einem Getränk, einem Kaugummi, einer Zahnpasta oder einer pharmazeutischen Zubereitung, dadurch gekennzeichnet, dass man zu dem genannten Artikel eine Verbindung der Formel (I), welche im Anspruch 1 definiert ist, in einer Konzentration, welche zwischen 20 ppb und 5 ppm des Gesamtgewichtes des Artikels beträgt, zusetzt.

12. 3-(4-Methylhexyl)pyridin.
